(19) Europäisches Patentamt
European Patent Office
Office européen des brevets

(11) Numéro de publication: **0 409 730 A1**

# (12) DEMANDE DE BREVET EUROPEEN

(21) Numéro de dépôt: **90402077.3**

(51) Int. Cl.5: **C12N 15/53**, C12N 15/82

(22) Date de dépôt: **18.07.90**

(30) Priorité: **19.07.89 FR 8909707**

(43) Date de publication de la demande: **23.01.91 Bulletin 91/04**

(84) Etats contractants désignés: **AT BE CH DE DK ES FR GB GR IT LI LU NL SE**

(71) Demandeur: **INSTITUT NATIONAL DE LA RECHERCHE AGRONOMIQUE**
**145, rue de l'Université**
**F-75341 Paris Cédex 07(FR)**

(72) Inventeur: **Daniel-Vedele, Françoise**
**7 rue Le Nôtre**
**F-78290 Bois D'Arcy(FR)**
Inventeur: **Caboche, Michel**
**5 rue de Thimerais**
**F-78310 Maurepas(FR)**

(74) Mandataire: **Warcoin, Jacques et al**
**Cabinet Régimbeau 26, avenue Kléber**
**F-75116 Paris(FR)**

## (54) Vecteur de clonage et d'expression du gène de la nitrate réductase de tomate.

(57) La présente invention a pour objet le gène de structure de la Nitrate Réductase de tomate, ainsi que des vecteurs de clonage et d'expression dudit gène et un procédé pour améliorer l'assimilation des nitrates dans les plantes, caractérisé en ce qu'on introduit dans des cellules de ces plantes une séquence d'ADN ou un vecteur selon l'invention dans des conditions permettant sa réplication et son expression.

EP 0 409 730 A1

La présente demande constitue un développement des demandes de brevet FR 2.610.948 et EP 283338 auxquelles on pourra se référer utilement pour la meilleure intelligence de la présente invention.

La présente invention concerne en effet le clonage du gène de structure de la Nitrate Réductase de tomate.

Dans les demandes de brevet FR 2.610.948 et EP 283338, un ADN complémentaire (ADNc) issu de l'ARN messager (ARNm) de la Nitrate Réductase de tabac, a été cloné. Les ADNc isolés représentaient plus de 50 % de l'ARNm de la Nitrate Réductase de tabac.

La présente invention a pour objet une séquence d'ADN codant pour la Nitrate Réductase de tomate.

On a découvert selon l'invention que les Nitrates Réductase des différentes espèces dicotylédones présentent une grande homologie comme il sera explicité ci-après, et notamment, la Nitrate Réductase de tomate présente environ 90 % d'homologie avec la Nitrate Réductase de tabac et la séquence d'ADN codant la Nitrate Réductase de tomate présente une homologie de plus 80 % (environ 81 %) avec la séquence d'ADN codant la Nitrate Réductase de tabac.

La présente invention a pour objet un vecteur de clonage selon l'une des revendications 1 à 6 du brevet principal, caractérisé en ce qu'il comporte une séquence d'ADN codant pour la Nitrate Réductase de tomate.

De préférence, un vecteur de clonage du gène de structure de la Nitrate Réductase de tomate selon l'invention comportera une séquence codante complète des 911 aminoacides de la Nitrate Réductase de tomate telle que représentée à la figure 1.

En particulier, on peut citer la séquence codante d'ADN telle que représentée en majuscule à la figure 1.

Il doit être bien entendu que l'invention a également pour objet toute séquence d'ADN équivalente, c'est-à-dire qui diffère de la séquence mentionnée ci-dessus seulement par une ou plusieurs mutations neutres, c'est-à-dire dont le changement ou la substitution de nucléotides en cause n'affecte pas la séquence primaire de la protéine résultante.

L'invention a également pour objet un vecteur de clonage du gène de structure de la Nitrate Réductase de tomate constituée par de l'ADNc de l'ARNm de la nitrate réductase de tomate.

Enfin, la présente invention concerne toutes séquences d'ADN complémentaires aux séquences mentionnées ci-dessus, en particulier qui présentent une homologie suffisante avec la séquence d'ADNc complémentaire de l'ARNm de la nitrate réductase de tomate, de telle sorte qu'elles s'hybrident avec ladite séquence d'ADNc à 80 dans des conditions stringentes.

A titre de vecteur de clonage comprenant le gène de structure de la nitrate réductase de tomate selon l'invention, on peut citer les vecteurs comprenant une séquence d'ADN contenant au moins une origine de réplication telle que des plasmides, des cosmides, des bactériophages, des virus, etc. On utilisera de préférence toutefois des plasmides.

De préférence, la séquence d'ADN codant pour la Nitrate Réductase de tomate sera associée à une séquence régulatrice appropriée pour sa transcription et sa traduction (ci-après régulon) tels que des promoteurs, y compris des codons start et stop, des "enhancer", des opérateurs. Les moyens et méthodes pour identifier et sélectionner ces promoteurs sont bien connus de l'homme du métier.

Les séquences d'ADN codant pour la Nitrate Réductase de tomate peuvent être introduits dans des cellules de plantes selon des mécanismes connus tels que par l'intermédiaire du plasmide Ti d' Agrobactérium tumefaciens , ou par transferts directs de gènes tels que par la méthode d'électroporation, ceci pour favoriser la faculté naturelle des plantes à assimiler les nitrates. On pourra utiliser avantageusement dans ce cas, mais non obligatoirement le régulon constitutif de la Nitrate Réductase.

Un vecteur de clonage selon l'invention est donc caractérisé en ce qu'il comporte en outre la séquence d'ADN précédent la séquence codante du gène de structure de la Nitrate Réductase de tomate, notamment telle que représentée à la figure 1, cette séquence comportant les éléments d'expression et des régulations (régulon) dudit gène de la Nitrate Réductase de tomate. Il s'agit alors d'un vecteur d'expression.

En particulier, un vecteur de clonage et d'expression selon l'invention est caractérisé en ce qu'il comporte la séquence d'ADN comprise entre la position 1 et 568, ou 232 et 483 du gène de la Nitrate Réductase de tomate de la figure 1.

Les vecteurs selon l'invention peuvent donc comporter des modes de réalisation particuliers de l'invention, une séquence d'ADN chosie parmi les séquences tirées de la figure 1 suivantes :

- les séquences de la position 1 ou 569 à 5309,
- les séquences de la position 1 ou 569 à 4658,
- la séquence de la position 569 à 4658 privée des introns représentés en lettres minuscules.

Alternativement, les séquences d'ADN codant pour la Nitrate Réductase de tomate peuvent être utilisées pour transformer des microorganismes ou des cellules eukaryotiques pour cloner et produire l'enzyme Nitrate Réductase de tomate.

La présente invention a enfin pour objet un procédé pour améliorer l'assimilation des nitrates dans les plantes, caractérisé en ce qu'on introduit dans les cellules desdites plantes un fragment d'ADN codant pour la Nitrate-Réductase de tomate selon l'invention, dans des conditions permettant sa réplication et son expression.

D'autres avantages et caractéristiques de la présente invention apparaîtront à la lumière de la description qui va suivre en référence aux figures 1 à 4.

La figure 1 (figures 1-A à 1-F) représente la sequence d'ADN complète du gène nia de tomate et séquence protéique prévue. Les séquences non codantes et codantes sont écrites respectivement en lettre miniscules et majuscules. Les séquences consensus sont soulignées et les trois premiers nucléotides présumés de l'ARNm sont en caractère gras.

La figure 2 représente la courbe G + C du gène nia de la tomate. Le teneur en G + C est calculée sur une fenêtre de 51 pb, par étapes de 20 pb. La structure du gène (voir figure 4) a été dessinée à la même échelle.

La figure 3 représente la comparaison de la structure des gènes nia de tomate et de tabac. Les régions 3' non traduites se terminent au premier signal de polyadénylation après le codon de terminaison.

La figure 4 (figures 4-A à 4-D) représente l'alignement des séquences protéiques entre NR de différentes espèces de plantes supérieures et protéines redox correspondantes. NR de tomate (ce travait), NR de nia2 de tabac (Vaucheret et al., 1989), NR2 d' Arabidopsis (Crawford et al., 1988), NR1 d' Arabidopsis et NR d'orge (Cheng et al, 1988), NADH:NR de maïs (Campbell et al., 1988b), sulfite oxydase de rat (Crawford et al., 1988), domaine catalytique de cytochrome b5 réductase humaine (Yubisui et al., 1984). Les positions où se trouvent deux résidus identiques ou plus sont encadrées. Au-dessus de l'alignement sont montrées les positions des introns (numéros 1, 2, 3), les limites des domaines (lettres a à h des cases inférieures) comme utilisé dans le tableau 2, les onze résidus conservés dans le domaine de liaison du hème et la lysine et la cystéine "essentielles" dans le domaine FAD/NADH (*).

## 1. Introduction

La nitrate réductase est une enzyme clé du métabolisme de l'azote dans les plantes supérieures. On a cloné et caractérisé le gène de structure de la nitrate réductase de la tomate. L'expression de ce gène est régulée par la lumière et l'apport azoté. Ce gène est long de 5 kpb. Il code pour une chaîne polypeptidique longue de 911 acides aminés, trois introns étant intercalés dans la séquence codante. La comparaison de ce gène avec les gènes de structure de la nitrate réductase du tabac montre une structure hautement conservée, y compris la position des introns. Trois domaines hautement conservés, un domaine de liaison du cofacteur molybdène, un domaine du hème et un domaine FAD/NADH se trouvent dans la séquence codante. La partie N-terminale de la protéine est moins conservée et contient un groupe de 8 résidus acides.

Le nitrate est la plus importante source d'azote pour les plantes supérieures. Le nitrate est absorbé par les racines, transporté dans divers tissus de la plante, puis réduit en ammoniaque en deux étapes. La première étape exige l'enzyme Nitrate Réductase (NR) qui catalyze la réduction du nitrate en nitrite dans le cytoplasme. Dans une seconde étape, le nitrite est ensuite réduit dans le chloroplaste par la Nitrite Réductase. La réduction du nitrate est considérée comme une étape de contrôle majeure dans l'assimilation du nitrate et elle a été étudiée en détail chez les plantes supérieures (Wray, 1986). La NR est un homodimère portant trois cofacteurs, à savoir FAD, cytochrome $b_{557}$ et un cofacteur molybdoptérine (Campbell, 1988a).

La régulation de la NR dans les plantes semble être très complexe et il a été montré que de nombreux facteurs tels que lumière, nitrate, ammonium et régulateurs de croissance influencent le niveau d'activité de la NR (Beevers and Hageman, 1983). L'isolement de clones d'ADNc codant pour l'apoprotéine NR a permis l'étude de cette régulation sur le plan moléculaire (Cheng et al., 1986 ; Crawford et al., 1986 ; Galangau et al., 1988). Les deux gènes nia du tabac ont été clonés. Leurs séquences nucléotidiques ont été obtenues récemment et la régulation de leur transcription a été étudiée dans différentes conditions physiologiques.

Le tabac a un génome complexe et il est peu caractérisé génétiquement et physiologiquement. Sur la base d'un criblage de 19 espèces de plantes différentes pour la présence de gènes nia homologues de notre sonde d'ADNc (par transfert de Southern - Southern blotting), on a choisi d'étudier le gène nia de la tomate et son expression. La tomate est une espèce diploïde vraie très bien connue sur une base génétique (rick, 1980). La tomate a été largement utilisée comme modèle pour des études agronomiques et la nutrition minérale de cette espèce est bien caractérisée. Par opposition au tabac, la tomate est également un modèle intéressant pour l'étude de l'influence de la nutrition minérale sur la maturation des fruits. Finalement l'isolement du gène nia de la tomate donne la possibilité de réintroduire ce gène dans un mutant de tabac hétérologue (Muller and Grafe, 1978) ou dans un mutant de Nicotiana plumbaginifolia déficient en NR (Gabard et al., 1987). Des mutants de tomate déficients en NR ont été récemment isolés (M Koorneef, communication personnelle) et il existe donc la possibilité d'étudier l'expression de ce gène réintroduit dans le génome de la tomate. Dans cette demande, on présente l'isolement du gène nia de la tomate et sa caractérisation par analyse de cartographie de restriction et séquençage nucléotidique. La séquence d'acides aminés de la protéine NR de la tomate, déduite de la séquence nucléotidique du gène, est comparée aux séquences de protéines NR déjà connues dans les plantes.

## 2. Résultats

### 2.1 Analyse Southern des gènes de la nitrate réductase

On a étudié le degré d'hybridation croisée entre les gènes nia de différentes espèces de plantes et notre sonde d'ADNc de la NR. Les ADN génomiques ont été digérés par Hind III et des hybridations par Southern blot et des lavages ont été effectués dans des conditions peu "stringentes" (voir Matériel et Méthodes) permettant l'hybridation stable des séquences nucléotidiques partageant au moins 70 % d'homologie. Les ADN génomiques correspondant à environ 5 x $10^6$ génomes de chaque espèce ont été utilisés pour ces expériences. On a utilisé comme sonde un clone d'ADNc du tabac codant pour une partie de la région carboxy terminale de la protéine (Calza et al., 1986).

Dix-espèces de plantes appartenant à sept familles ont été étudiées (tableau 1). Les familles de Solanacées (5 espèces) et de Cucurbitacées (1 espèces) étaient caractérisées par un taux constant d'homologie. Cette homologie relativement élevée suggère que les séquences nucléotidiques des gènes nia sont bien conservées parmi ces espèces. Par contre, on a observé une hétérogénéité surprenante dans trois familles, les Crucifères, les Composées et les Légumineuses. Par exemple, cette dernière famille présentait 3 niveaux d'hybridation : on n'a pu détecter aucun signal avec l'ADN génomique extrait de Pisum sativum ou Lotus coniculatus , mais on a obtenu un puissant signal d'hybridation avec l'ADN extrait de Trifolium pratense . Medicago sativa et Phaseolus vulgaris ont donné des signaux intermédiaires. Beta vulgaris et Zea mays représentaient respectivement les familles des Chénopodiacées et des Graminées. Dans ces deux cas, aucun signal n'a été obtenu dans nos conditions expérimentales.

Parmi les génomes de plantes montrant une intensité élevée de signaux d'hybridation, on a noté une variation dans le nombre de fragments s'hybridant de façon croisée à la sonde de NR. Des bandes multiples ont été obtenues dans le cas de Nicotiana tabacum , Medicago sativa et Trifolium patense . La complexité de ce schéma peut s'expliquer d'une part par l'amphidiploïdie du génome, comme observé précédemment pour le tabac (Vaucheret et al., 1989). D'autre part, la luzerne et le trèfle appartiennent à la famille des Légumineuses et la présence de trois formes de NR, NAD(P)H ou NADH dépendantes, est déjà connue dans une plante de cette famille, à savoir le soja (Campbell, 1988b). On peut imaginer que dans des conditions d'hybridation peu "stringentes" on a détecté les gènes correspondant à ces enzymes.

### 2.2 Isolement du gène de la NR de la tomate

Les expériences de Southern blot effectuées sur l'ADN de tomate digéré par Eco RI (données non présentées) en utilisant l'ADNc de tabac comme sonde marquée radioactivement, ont révélé un fragment unique long de 6,5 Kb dans ce génome. Nous avons construit une banque génomique partielle avec l'ADN de tomate digéré complètement par Eco RI, fractionné par tailles sur gradients de saccharose et lié à l'ADN de lambda gt WES digéré par Eco RI. 250 000 clones recombinants ont été criblés par des hybridations in situ avec la sonde du tabac dans des conditions peu "stringentes" (voir Matériel et Méthodes) et 6 clones indépendants ont été isolés. Tous ces clones contenaient le même fragment d'ADN de tomate long de 6,5 kb. L'analyse par séquençage (voir plus loin) a montré que ce fragment ne contenait pas le gène nia de tomate entier. Afin d'isoler l'extrémité 5′ du gène, nous avons contruit une nouvelle banque génomique avec l'ADN de tomate totalement digéré par Hind III, fractionné par taille sur gradients de saccharose et lié à l'ADN de lambda NM 1149, digéré par Hind III. Parmi 300 000 clones recombinants, criblés par hybridation in situ avec le fragment de 6,5 Kb de tomate dans des conditions hautement "stringentes", 16 clones positifs ont été détectés. Cinq d'entre eux ont été caractérisés de façon plus approfondie et ils contenaient un fragment long de 7 Kb ayant en commun une région de 1,5 Kb avec le fragment Eco RI isolé précédemment.

2.3 Analyse de la séquence nucléotidique du gène de la NR de la tomate

Afin de déterminer la séquence nucléotidique du gène nia entier de tomate, on a sous-cloné des fragments de restriction dans des vecteurs Bluescript abritant un "polylinker" (lieur multisite) avec des sites de clonage multiples dans chaque direction. Ces caractéristiques du vecteur ont permis de déterminer, par des délétions unidirectionnelles (voir Matériel et Méthodes) la séquence nucléotidique sur les deux brins d'un fragment long de 5309 pb, contenant le gène nia entier avec les régions adjacentes 5′ et 3′ non codantes. Cette séquence est présentée à la figure 1. La taille et les positions des introns ont été en outre déduites par analyse par ordinateur de la séquence et de la position des cadres de lecture ouverts supposés par comparaison avec l'ADNc de tabac. Les trois introns commencent par le dinucléotide GT et finissent par le dinucléotide AG, comme observé régulièrement aux jonctions intron/exon des gènes eucaryotes. La teneur moyenne en A + T de ces régions est de 73 %, alors que la teneur en A + T du cadre de lecture ouvert est de 56 %. La courbe de la teneur en G + C en fonction de la position des nucléotides dans la séquence du gène nia est représentée sur la figure 2. Un accord presque parfait entre la position des introns et la teneur réduite en G + C est observée. Le changement dans la teneur en G + C est détecté exactement à la limite entre exons et introns. Un niveau aussi élevé de la séquence A + T dans les introns semble être un caractère commun aux gènes des plantes et il est supposé jouer un rôle dans l'épissage spécifique des introns des plantes par le mécanisme des plantes (Wiebauer et al., 1988).

Un hexanucléotide AATAAA, souligné sur la figure 1, est situé 44 pb après l'extrémité du dernier exon. Cette séquence cadre parfaitement avec la séquence consensus trouvée 10-30 bases en amont du site de polyadénylation dans la majorité des ARNm (Joshi, 1987a) et elle est absolument nécessaire pour la polyadénylation (Wickens et Stephenson, 1984). Récemment, J. Wilusz et T. Shenk (Wilusz and Shenk, 1988) ont démontré la liaison d'une protéine nucléaire de 64 Kd avec des segments d'ARN qui comprennent ce motif.

Le site d'initiation de transcription a été déterminé par comparaison avec les promoteurs de NR de tabac sur lesquels des expériences d'extension d'amorce ont été effectuées (Vaucheret et al., 1989). Ce site a été localisé 48 pb en amont du codon d'initiation du gène de la tomate. Les trois premiers nucléotides CGT du transcrit ARNm de tomate (en caractères gras sur la figure 1) cadre avec la séquence consensus, PyPuPy, connue pour la plupart des gènes d'animaux. Dans la cellule eucaryote, l'initiation de transcription dépend de la reconnaissance d'un élément de la séquence TATA par l'ARN polymérase II. Un tel élément, une séquence TATATAAA, se trouve en position -33 du site d'initiation de la transcription, en accord avec la position de la plupart de ces éléments dans les cellules végétales (Joshi, 1987b).

Une CCAAT box incomplète, la séquence CAAT soulignée sur la figure 1, est localisée 72 pb en amont de l'élément TATA. Cette séquence est supposée moduler les niveaux de transcription dans les gènes d'animaux. De plus, A. Dorn et coll. (Dorn et al., 1987) ont récemment établi la multiplicité des protéines fixant la CCAAT box impliquées dans la régulation de l'expression des gènes de classe II du complexe d'histocompatibilité majeur de la souris.

Ces données sur les séquences apparaîtront dans les EMBL/GenBank/DDBJ Nucleotide Sequence Databases sous le numéro d'accession X14060.

2.4 Comparaison de trois gènes nia

Une homologie étendue a été trouvée entre les gènes nia de tomate et de tabac dérivés soit de Nicotiana sylvestris (Vaucheret et al., 1989) soit de Nicotiana tomentosiformis . Le cadre de lecture ouvert est interrompu par trois introns localisés au même site dans une séquence codante du tabac et de la tomate, mais se distinguant par la taille. Les introns du gène de la tomate sont plus petits que ceux des gènes du tabac. Les introns 1, 2 et 3 dans la tomate sont respectivement de 74, *846* et *437* pb comparés à 594, 1298 et 788 pb dans le gène du tabac de Nicotiana sylvestris par exemple (figure 3). Les régions 5′ et 3′ non traduites sont également plus petites dans le gène nia de la tomate que dans les gènes du tabac.

Les séquences nucléotidiques des exons sont très bien conservées entre ces deux gènes (81 %), alors que les séquences nucléotidiques des introns ont divergé plus fortement pendant l'évolution des espèces de tabac et de tomate.

La comparaison des promoteurs de la NR de tabac et de tomate révèle une homologie frappante dans une région longue de 250 pb, localisée dans le gène de la tomate entre les positions 233 et 483. Cette région, contenant les TATA et CAAT boxes, est à 75 % homologue d'un fragment localisé à la même position dans le gène du tabac.

2.5 Analyse de la séquence de la protéine NR de la tomate et comparaison avec les NR d'autres plantes supérieures

La séquence polypeptidique déduite de la NR de la tomate est longue de 911 acides aminés, longueur qui se situe entre celle de la NR2 de l'arabidospis (917) et celle de la NR du tabac (904). La séquence de la tomate montre une analogie élevée (90,5 %) avec les deux séquences de NR du tabac qui sont elles-mêmes homologues à 98 %. Les NR de tomate et de tabac ont été comparées à d'autres séquences complètes ou partielles de NR de plantes supérieures (figure 4), les deux isoformes de l'arabidospis NR1 (Cheng et al., 1988) et NR2 (Crawford et al., 1988) et les NR du maïs (Campbell, 1988b) et de l'orge (cheng et al., 1986). Le score d'homologie se situe entre 76 % et 65 %, suivant la distance phylogénétique, ce qui signifie que la NR est très bien conservée dans les plantes supérieures.

La Nitrate Réductase, une enzyme à trois centres redox, semble s'être développée à partir de la fusion de gènes entre des séquences codant pour des protéines à un centre redox. L'homologie des séquences NR avec deux de ces protéines offre la possibilité de lier séquence et fonction de la moitié C-terminale du polypeptide. L'homologie remarquablement élevée avec tous les membres de la superfamille du cytochrome b5 et avec la cytochrome b5 réductase (Calza et al., 1987) définit nettement les limites des régions hémique et FAD/NADH, qui sont séparés par une région charnière de 25/30 aa. Onze résidus, parmi lesquels se trouvent les deux histidines liant le hème, sont conservés dans le domaine hémique comme chez tous les membres de la superfamille du cytochrome b5 (Guiard and Lederer, 1979). L'on sait qu'un thiol catalytique joue un rôle essentiel dans les activités de liaison du NADH de la NR et de la déshydrogénase associée de même que dans l'activité de la cytochrome b5 réductase. Un bon candidat pour cette fonction est le résidu de cystéine conservé dans le domaine C-terminal, comme indiqué aussi par Crawford (Crawford et al., 1988), qui se situe de plus à la limite d'une longue séquence conservée, C GPPPMI (figure 4). Il existe cependant une différence, pour la cytochrome b5 réductase, entre l'homologie des séquences, qui milite en faveur de $Cys^{273}$, et l'expérience de marquage du N-éthylmaléimide où le $Cys^{283}$ a été marqué (Hackett et al., 1986), mais pour lequel il n'y a pas de résidu équivalent dans les séquences de NR. Un autre résidu critique de cytochrome b5 réductase, dont on salt qu'il est impliqué dans la liaison de NaDH par l'intermédiaire d'un groupement amino, a été identifié comme $Lys^{110}$ (Hackette et al., 1988). Un résidu de lysine se trouve également en une position homologue au résidu $Lys^{110}$ dans chaque NR (figure 4). D'autres résidus essentiels, soit catalytiques (une tyrosine liant la flavine) soit impliqués dans la charge appariement avec le cytochrome b5, restent à identifier dans la séquence de cytochrome b5 réductase (Hackett et al., 1988) et également, très probablement, dans le domaine flavodéshydrogénase de la NR.

Les résidus essentiels cystéine et lysine sont assez éloignés sur la séquence, mais devraient être proches dans la structure tertiaire. Cela implique qu'il n'y a pas de domaines de liaison de FAD et NADH séparés, comme on le trouve pour de nombreuses flavoenzymes telles que la glutathion réductase. Dans cette famille d'enzymes, un repli du dinucléotide de Rossmann se trouve dans chacun de ces domaines. Il n'y a aucune indication d'une telle structure supersecondaire dans la famille RN/cytochrome b5 réductase.

Le(les) domalne(s) Moco/$NO_3$ devrait(ent) occuper la moitié N-terminale de la chaîne polypeptidique. Deux courts peptides, obtenus à partir du domaine molybdoptérine de la sulfite oxydase, semble être significativement homologues des séquences de NR dans la partie N-terminale (Crawford et al., 1988), ce qui confirme cette hypothèse. Toutefois, son étendue précise reste inconnue étant donné qu'aucune homologie évidente avec une autre molybdoprotéine complètement séquencée n'a été trouvée, même avec la xanthine déshydrogénase de la drosophile ou la nitrate réductase d' E. coli , dont on sait qu'elles fixent un cofacteur très semblable. Les trois sites des introns s'avèrent être localisés dans cette région, mais il n'y a aucune indication qu'ils séparent des unités fonctionnelles comme ils le font souvent. La séquence N-terminale réelle, jusqu'à la position 80, contient un groupe polyacide de 8 aa, et est moins bien conservée que n'importe quelle partie de la séquence de NR (tableau 2). Cela suggère que cette séquence n'est pas impliquée dans le processus catalytique, mals jouerait plus probablement un rôle structural ou régulateur. L'homologie n'est pas uniformément distribuée sur toute la séquence restante, le domaine FAD/NADH semblant s'être développé plus librement que le(les) domaine(s) Moco (tableau 2).

En examinant la distribution des positions homologues dans les alignements des séquences NR (figure 4), les positions les mieux conservées semblent se regrouper dans les segments qui devraient porter une importance structurale et/ou fonctionnelle, séparés par de cours résidus variables et habituellement hydrophiles qui se retrouveront probablement à la surface de la molécule de NR et sont par conséquent des candidats pour être des épitopes spécifiques d'espèces pour des anticorps monoclonaux (Chérel et al., 1986).

Tableau 1

| Hybridations croisées entre gènes de NR parmi 19 espèces de plantes | | |
|---|---|---|
| Famille | Espèce | Signaux d'hybridation |
| Solanaceae | Nicotiana tabacum | + + + |
| | Nicotiana plumbaginifolia | + + + |
| | Nicotiana glauca | + + + |
| | Solanum tuberosum | + + + |
| | Lycopersicon esculentum | + + + |
| Cucurbitaceae | Cucumis sativus | + + + |
| Leguminosae | Lotus corniculatus | - |
| | Pisum sativum | - |
| | Phaseolus vulgaris | + |
| | Medicago sativa | + |
| | Trifolium pratense | + + + |
| Cruciferae | Brassica oleacera | - |
| | Brassica napus | - |
| | Sinapis alba | + + |
| | Arabisopsis thaliana | + + + |
| Compositae | Helianthus annuus | + |
| | Cichorium intybus | + |
| | Lactuca sativa | + + |
| Chenopodiace | Beta vulgaris | - |
| Graminae | Zea mays | - |

Tableau 2

| Comparaison des séquences de domaines de NR | | | | |
|---|---|---|---|---|
| Tomate Domaines NR | Tabac nial | Tabac nia2 | Arabidopsis NR2 | Maïs NADH:NR |
| Séq. entière | 89,7 | 90,5 | 76,0 | (73,5)* |
| a:N-terminal | 69,9 | 72,3 | 37,2 | - |
| b:MoCol | 92,6 | 92,6 | 79,0 | - |
| c:MoCo2 | 96,1 | 96,1 | 88,6 | 89,8 |
| e:heme | 91,7 | 95,2 | 85,7 | 76,7 |
| f:charnière | 86,2 | 86,2 | 71,4 | 44,8 |
| g:FAD | 90,2 | 91,7 | 75,7 | 63,2 |
| h:NADH | 88,0 | 88,7 | 71,6 | 65,4 |
| Les limites des domaines sont désignées par des lettres minuscules (a à h) sur l'alignement des séquences protéiques sur la figure 4. L'homologie est calculée en pour cent des concordances parfaites avec la séquence de tomate utilisé comme référence. | | | | |

* la séquence NR de maïs est une séquence C-terminale partielle

## 3. Matériel et méthodes

### 3.1 Matériel végétal et extraction d'ADN génomique

Les plantes de tomate ( Lycopersicon esculentum cv Manapal) ont été cultivées en serre jusqu'à ce qu'elles aient développé 6-7 feuilles. A ce stade, les feuilles ont été excisées, mélangées, pesées et congelées immédiatement dans l'azote liquide. Des échantillons ont été conservés à -80° C jusqu'à l'analyse ultérieure.

L'ADN génomique a été isolé par la méthode de Dellaporta (Dellaporta, 1983) et a été purifié par centrifugation en gradient de chlorure de césium.

### 3.2 Expériences de Southern bloting (transfert de Southern)

Les ADN totaux ont été digérés par l'enzyme de restriction Hind III, fractionnés par taille sur des gels d'agarose et transférés sur des membranes de nitrocellulose (Amersham). La sonde d'ADNc de NR dérivée du tabac, pBMC102010 (Calza et al., 1987) a été marquée radioactivement par alpha$^{32}$p-dCTP (Amersham) par "nick translation" (déplacement de coupure) à 5 x $10^8$ cpm/µg et utilisée à une concentration de $10^6$ cpm/ml de la solution d'hybridation. Les hybridations dans des conditions faiblement "stringentes" ont été effectuées à 55° C dans 5 x SSPE, 2 x solution de Denhart, SDS 0,1 % (p/v) (1 x SSPE = 0,15 M de NaCl, 8,8 mM de $NaH_2PO_4$, 1 mM d'EDTA pH 7,5). Les lavages ont été effectués 3 x 10 min à température ambiante et 2 x 30 min à 55° C dans 2 x SSC, SDS 0,1 % (1 x SSC = 0,15 M de NaCl, 3 mM de citrate de Na). Des conditions hautement "stringentes" ont été obtenues en augmentant la température des hybridations et des lavages jusqu'à 65° C.

### 3.3 Construction de banques génomiques de tomate

L'ADN de tomate digéré complètement par les enzymes de restriction Eco RI ou Hind III, a été

fractionné par taille en gradients de 5-40 % de saccharose. Des expériences de Southern blotting ont été effectuées sur des fractions contenant l'ADN de la taille prévue et les fractions montrant le signal d'hybridation le plus puissant ont été choisies pour la construction des banques. L'ADN de ces fractions a été lié au lambda gt WES digéré par Eco RI (Stratagene Inc.) ou au lambda NM1 149 digéré par Hind III (Murray, 1983). L'encapsidation in vitro des molécules liées a été obtenue à 22°C pendant 2 heures en utilisant des extraits du commerce (Gigapack, Clontechlabs, CA). Les phages recombinants ont été cultivés sur des souches E. coli LE 392 pour lambda gt WES ou POP13 pour lambda NM1149. Un rendement de 5 x $10^5$ clones recombinants/µg d'insert d'ADN a été habitellement obtenu. Des hybridations in situ de phages recombinants utilisant soit la sonde ADNc de tabac soit le premier fragment d'ADN de tomate isolé ont été effectuées dans des conditions respectivement faiblement ou hautement "stringentes" comme décrit dans les expériences de Southern blotting (transfert de Southern). Les phages positifs ont été purifiés et obtenus par 2 autres cycles d'hybridations in situ .

3.4 Séquençage nucléotidique

Les vecteurs Bluescript (Stratagene, Inc.), portant des sites de clonage multiples dans un polylinker (lieur multisite) et la région intergénique du simple brin du phage M13 ont été utilisés en tant que système de séquençage d'ADN. Les fragments d'ADN de taille appropriée devant être séquencés ont été générés dans le vecteur par des délétions en utilisant des digestions par ExoIII et nucléase de haricot-mungo. Les réactions de séquençage ont été effectuées par la méthode du didésoxynucléotide de Sanger (Sanger, 1977), en utilisant l'enzyme séquénase pour la réaction de polymérisation.

## 4. Discussion

La réduction du nitrate est considérée comme étant l'étape contrôlant l'assimilation du nitrate par les plantes supérieures. Des ADNc partiels ou complets codant pour l'apoprotéine NR ont été isolés de l'orge (Cheng et al., 1986), de la courge (Crawford et al., 1986), du tabac (Calza et al., 1987), de l' Arabidopsis (Crawford et al., 1988) et du maïs (Campbell, 1988 b). Récemment, les deux gènes homéologues du tabac ont été clonés et leur séquence nucléotidique a été déterminée (Vaucheret et al., 1989). Dans cette demande, on décrit pour la première fois la séquence complète du gène codant pour l'apoprotéine NR d'une plante ayant un intérêt agronomique, à savoir la tomate.

Dans plusieurs espèces de plantes diploïdes y compris l'orge (Kleinhofs et al., 1988) et Arabidopsis thaliana (Braaksma and Fenstra, 1982), plusieurs gènes codent pour la NR. Il est assez surprenant que la situation soit remarquablement différentes chez les espèces de solanacées. L'analyse génétique des gènes nia dans le tabac (Muller and Grafe, 1978) et Nicotiana plumbaginifolia (Gabard et alk., 1987) a montré l'existence uniquement de deux gènes nia dans le tabac, une espèce amphiploïde, et d'un gène nia dans Nicotiana plumbaginifolia . L'expérience du Southern blotting selon l'invention suggère que dans la tomate, la NR est codée par un seul gène. En conséquence, les espèces solanacées semblent convenir idéalement à l'étude de l'expression de NR. Les genes nia de la famille des Solanacées présentent un haut degré d'homologie avec la séquence du tabac. Le degré variable d'homologie des gènes NR des familles de Légumineuses, de Crucigères et de Composées avec le gène du tabac reflète peut-être une évolution particulière des gènes nia , adaptée à chaque espèce de plante. Le profil complexe d'hybridation observé chez plusieurs espèces de plantes semble être dû plus probablement soit à des formes multiples de NR, NADH ou NADPH dépendantes, soit au niveau de ploïdie de certaines génomes de plantes plutôt qu'à la présence de pseudo-gène étant donné que de tels pseudo-gènes ne semblent pas exister dans les génomes de tabac et de tomate.

L'analyse du clonage moléculaire et des séquences nucléotidiques révèle que le gène codant pour l'apoprotéine NR de la tomate présente tous les caractères d'un gène eucaryote fonctionnel. Les séquences consensus, comme les CAAT et TATA boxes (boîtes) ou les signaux de polyadénylation se trouvent aux positions appropriées le long de la séquence nucléotidique. La comparaison de ce gène et du gène nia de Nicotiana sylvestris décrit précédemment (Vaucheret et al., 1989) révèle un haut degré d'homologie dans leur organisation structurale. Les trois introns sont localisés aux mêmes sites, mais sont plus petits dans le gène de la tomate. Il est possible que cela reflète la taille génomique relative de la tomate ($1{,}9.10^9$ pb) et du tabac ($1{,}5.10^{11}$pb). Etant donné que la régulation de l'expression du gène de NR semble être analogue dans les deux espèces de plantes (Galangau et al., 1988), il est possible de s'imaginer que la région homologue de 250 pb des promoteurs de NR du tabac et de la tomate est impliquée dans la régulation des gènes médiée par la lumière ou le nitrate. Aucune région évidente d'homologie n'a été trouvée en amont ou en aval de ces séquences. La structure secondaire trouvée dans la séquence "leader" (de tête) du gène nia-2 du tabac (Vaucheret et al., 1989) n'est pas présente dans le gène de la tomate et il est possible par conséquent qu'elle ne soit pas en rapport avec la régulation de la traduction de l'ARNm de NR.

Une comparaison des séquences d'acides aminés de NR de différentes espèces conduit à la constatation de séquences conservées qui sont supposées être essentielles pour la structure et les activités de la protéine NR. La région N-terminale longue de 80 aa semble avoir divergé dans ces espèces. Cependant, une région centrale longue de 220 aa, impliquée probablement dans la liaison de la molybdoptérine et/ou la réduction de $NO_3$, est très bien conservée parmi les NR des plantes supérieures.

Il a été trouvé que le cytochrome b5 et la cytochrome b5 réductase sont homologues des domaines hémique et FAD/NADH de la NR. D'après les résultats expérimentaux obtenus sur la NR des chlorelles (Solomonson et al., 1986) et, plus récemment, sur la NR d'épinard (Kubo et al., 1988), il existe de bonne preuves selon lesquelles ces domaines sont de vraies unités structurales. En effet, par une protéolyse limitée de l'enzyme des épinards par la trypsine ou la protéase de S. aureus V8, la liaison covalente entre les domaines MoCo et hémique ou entre les domaines hémique et FAD est rompue. De plus, les fragments protéolytiques conservent une activité catalytique, soit la ferricyanure réductase pour le domaine FAD de 28kDA, soit la NADH:cytochrome c réductase pour le fragment de 45 KDA (domaines hème + FAD). Ce résultat est en parfait accord avec les données sur les séquences, où deux régions charnières entre chaque domaine ont été prévues (régions d et f, où les résidus sont moins bien conservés, hydrophiles et où il n'y a aucune indication d'une structure secondaire ordonnée). La protéolyse observée a lieu très probablement dans ces régions charnières, où se trouvent des résidus basiques ou acides appropriés pour chaque protéase. L'expérience de clivage protéolytique montre également qu'une structure covalente est une condition absolument nécessaire pour une activité NADH:NR, l'interaction entre domaines étant probablement trop faible pour maintenir une structure quaternaire active.

Etant donné que le cytochrome b5 et la cytochrome b5 réductase sont des protéines redox universelles, leurs relations structure-fonction sont très bien étudiées. L'on espère que les constatations faites sur leur mécanisme catalytique s'appliqueront par homologie à la nitrate réductase. Inversement, la nitrate réductase étant un analogue covalent du complexe xytochrome b5-cytochrome b5 réductase pourrait peut-être aider à élucider comment ces protéines réagissent entre elles.

## 5. Fonctionalité du gène de structure de la NR de tomate

La fonctionalité du gène de structure de la Nitrate Réductase de tomate a été testée par des expériences de complémentation d'un mutant Nitrate Réductase déficient de Nicotiana plumbaginifolia .

Le gène de structure de la Nitrate Reductase de tomate a été cloné sous le contrôle de son propre promoteur dans un vecteur binaire d' Agrobactérium tumefaciens , le plasmide Bin 19 (Bevan, M. (1896) Binary Agrobacterium vectors for plant transformation). Nucleic acids research 12 (22) 8711-8721). Des explants foliaires du mutant E 23 de Nicotiana plumbaginifolia ont été infectés par une souche d' Agrobacterium tumefaciens portant ce plasmide Bin 19 recombinant.

Environ 150 cals ont été obtenus sur milieu contenant de la kanamycine, dont le gène de résistance est porté par le plasmide bin 19. 80 % d'entre eux donnent naissance à des plantes transgéniques dont le phénotype "transformé" est mis en évidence par leur résistance à la kanamycine et leur aptitude à croître sur nitrate comme seule source d'azote.

Actuellement 18 plantes transgéniques sont élevées en serre et poussent sur leurs propres racines. Des premiers tests d'activité Nitrate Réductase ont été effectués sur 5 d'entre elles et les résultats ont été comparés à l'activité Nitrate Réductase d'une plante Nicotiana plumbaginifolia sauvage.

| Plantes | Activité Nitrate Réductase |
|---|---|
| Type sauvage | 100 % |
| 3-1-C | 3 % |
| E-2-4 | 7 % |
| B-2-X-2 | 10 % |
| B-2-Y-2 | 10 % |
| 3-F-1 | 23 % |
| Mutant E 23 | <0.1 % |

LITTERATURE CITEE

**Beevers, L. and Hageman, R.H.** (1983). Uptake and reduction of nitrate:bacteria and higher plants. In Encyclopedia of Plant Physiology, A. Lauchli and R.L. Bieleski, eds (Springer, New-york) **Vol.15A** pp 351-375.

**Braaksma,F.J. and Feenstra, W. J.** (1982). isolation and characterization of nitrate reductase deficient mutant of *Arabidopsis thaliana*.. Theor. Appl. Genet. **64,** 83-90.

**Calza, R., Huttner, E., Vincentz, M., Rouzé, P., Galangau, F., Vaucheret, H., Chérel, I., Meyer,C., Kronenberger,J. and Caboche,M.** (1987). Cloning of DNA fragments complementary to tobacco nitrate reductase mRNA and encoding epitopes common to nitrate reductases from higher plants. Mol. Gen. Genet. **209,** 552-562.

**Campbell, W. H.** (1988a). Higher plant nitrate reductase: arriving at a molecular view. Curr. Top. Plant Biochem. Physiol. **7,**1-15.

**Campbell,W. H.** (1988b). Structure and synthesis of higher plant nitrate reductase. In Molecular and Genetic Aspects of Nitrate Assimilation, Wray, J. L. and Kinghorn J. R., eds (Oxford Univ. Press), in press.

**Cheng, C.H., Dewdney, J., Kleinhofs, A. and Goodman,H. M.** (1986). Cloning and nitrate induction of nitrate reductase mRNA. Proc. Natl. Acad. Sci. USA **83,** 6825-6828.

**Cheng, C. H., Dewdney, J., Nam, H. G., den Boer, B. G. W. and Goodman, M.** (1988). A new locus (NIA 1) in *Arabidopsis thaliana* encoding nitrate reductase. EMBO J. **7,** 3309-3314.

**Chérel, I., Marion-Poll, A., Meyer C. and Rouzé, P.** (1986). Immunological comparisons of nitrate reductases of different plant species using monoclonal antibodies. Plant physiol. **81,** 376-378

**Crawford, N. M., Campbell, W.H. and Davis, R. W.** (1986). Nitrate reductase from squash: cDNA cloning and nitrate regulation. Proc. natl. Acad. Sci. USA **93,** 8073-8076.

**Crawford, N. M., Smith, M., Bellissimo, D. and Davis, R. W.** (1988). Sequence and nitrate regulation of the *Arabidopsis thaliana* mRNA encoding nitrate reductase, a metalloflavoprotein with three functional domains. Proc. Natl. Acad. Sci. USA **85,** 5006-5010.

**Dellaporta, S. L., Wood, J. and Hicks, J. B.** (1983). A plant DNA minipreparation: version II. Plant. Mol. Biol. Reporter.**1**, 19-21.

**Dorn, A., Bollekens, J., Staub, A., Benoist, G. and Mathis, D.** (1987). A multiplicity of CAAT box-binding proteins. Cell. **50,** 863-872.

**Gabard, J., Marion-Poll, A., Chérel, I., Meyer, C., Muller, A. J. and Caboche,M.** (1987). Isolation and characterization of *Nicotiana plumbaginifolia* nitrate reductase deficient mutants: genetic and biochemical analysis of the nia complementation group. Mol. Gen. Genet. **209,** 596-606.

**Galangau, F., Daniel-Vedele, F., Moureaux, T., Dorbe, M. F., Leydecker, M. T. and Caboche, M.** - (1988). Expression of leaf nitrate reductase genes from tomato and tobacco in relation to light-dark regimes and nitrate supply. Plant Physiol. **88,** 383-388.

**Guiard, B. and Lederer, F.** (1979). The "cytochrome $b_5$ fold': structure of a novel protein superfamily. J. Mol. Biol. **135,** 639-650.

**Hackett, C.S., Novoa, W.B., Ozols, J. and Strittmatter, P.** (1986). Identification of the essential cysteine residue of NADH-cytochrome b5 Reductase. J. Biol. Chem. **261,** 9854-9857

**Hackett, C.S., Novoa, W.B., Read Kensil, C. and Strittmatter, P.** (1988). NADH binding to cytochrome b5 Reductase blocks the acetylation of lysine 110. J. Biol. Chem. **263,** 7539-7543

**Joshi, C. P.** (1987a). Putative polyadenylation signals in nuclear genes of higher plants: a compilation and analysis. Nucl. Acid. Res. **15,** 9627-9640.

**Joshi, C.P.** (1987b). An inspection of the domain between putative TATA box and translation start in 79 plant genes. Nucl. Acid. Res. **15,** 6643-6653.

**Kleinhofs, A., Warner, R. L., Hamat, H. B., Juricek, M., Huang, C. and Schnorr, K.** (1988). molecular genetics of barley and rice nitrate reductases. Curr. Top. Plant Biochem. and Physiol. **7,** 35-42.

**Kubo, Y., Ogura, N. and Nakagawa, H.** (1988). Limited preoteolysis of the nitrate reductase from spinach leaves. J. Biol. Chem. 263, 19684-19689.

**Muller, A. J. and Grafe, R.** (1978). Isolation and characterization of cell lines of *Nicotina tabacum* lacking nitrate reductase. Mol. Gen. Genet. **161,** 67-76.

**Murray, N. E.** (1983). Phage lambda and molecular cloning.In, Lambda II, Hendrix, R. W., Roberts,J. W., Stalh, F. W. and Weisberg R. A. ,eds (Cold Spring Harbor, New-York) pp 395-432.

**Ozols, J. and Strittmatter, P.** (1969). Correction of the amino acid sequence of calf liver cytochrome b5. J. Biol. Chem. **244,** 6617-6618.

**Rick, C. M.** (1980). Linkage map of the tomato (*Lycopersicon esculentum*). In Genetic maps, Brien, S., ed (NIH Bethesda). **Vol 1,**268-281.

**Sanger, F., Nicklen, DS. and Coulson, A. R.** (1977). DNA sequencing with chain termination inhibitors. Proc. Natl. Acad. Sci. USA **74,** 5463-5467.

**Solomonson, L. P., Barber, M. J., Robbins, A. P. and Oaks, A.** (1986). Functional domains of assimilatory NADH: nitrate reductase of *Chlorella*. J. Biol. Chem. **261,** 11290-11294.

**Vaucheret, H., Vincentz, M., Kronenberger,J., Caboche, M. and Rouzé, P.** (1989). Molecular cloning and characterization of the two homeologous genes coding for nitrate reductase in tobacco. Mol. Gen. Genet. In press.

**Wickens, M. and Stephenson,B.** (1984). The role of the conserved AAUAAA sequence in mRNA maturation: four AAUAAA points mutants prevent 3′ end formation. Science **226,** 1045-1051.

**Wiebauer, K., Herrero, J.J. and Filipowicz, W.** (1988). Nuclear pre-mRNA processing in plants: distinct modes of 3′-splice site selection in plants and animals. Mol. and Cell. Biol. **8,** 2042-2051.

**Wilusz, J., and Shenk, T.** (1988). A 64Kd nuclear protein binds to RNA segments that includes the AAUAAA polyadenylation motif. Cell. **52,** 221-228.

**Wray, J. L.** (1986). The molecular genetics of higher plant nitrate assimilation. In, A genetic Approach to Plant Biochemistry, A. D. Blonstein and P.J. King, eds (Springer, New-York) pp 101-157.

**Yubisui, T., Miyata, T., Iwanaga, S., Tamura, M., Yoshida, S., Takeshita, M. and Nakajima, H.** - (1984). Amino-acid sequence of NADH-Cytochrome b5 reductase of human erythrocytes. J. Biochem. **96,** 579-582.

## Revendications

1. Vecteur de clonage caractérisé en ce qu'il comporte une sequence d'ADN codant pour la Nitrate Réductase de tomate.

2. Séquence d'ADN utile notamment dans le vecteur de clonage selon la revendication 1, caractérisée en ce qu'il s'agit d'une séquence d'ADN codant pour les 911 aminoacides de la Nitrate Réductase de tomate représentés à la figure 1.

3. Séquence d'ADN selon la revendication 2, caractérisée en ce qu'elle consiste dans la séquence d'ADN complète codant pour la Nitrate Réductase de tomate représentée en majuscule à la figure 1.

4. Vecteur de clonage de la nitrate réductase de tomate, caractérisée en ce qu'il comporte une séquence d'ADN selon l'une des revendications 2 ou 3.

5. Vecteur de clonage du gène de structure de la Nitrate Réductase de tomate selon l'une des revendications 1 et 4, caractérisé en ce que la séquence d'ADN est de l'ADN complémentalre de l'ARN messager de la Nitrate Réductase de tomate obtenue par transcription inverse.

6. Vecteur de clonage et d'expression selon l'une des revendications 1 à 5, caractérisé en ce qu'il comporte en amont et en phase de lecture de ladite sequence d'ADN codant la Nitrate Réductase de tomate, une séquence d'ADN comportant des éléments d'expression et du régulation du gène de la Nitrate Réductase de tomate.

7. Vecteur de clonage et d'expression selon la revendication 6, caractérisé en ce qu'il comporte en outre la séquence d'ADN précédent la séquence codante du gène de structure de la Nitrate Réductase de tomate allant de la position 1 à 568 telle que représentée à la figure 1, cette séquence comportant les éléments d'expression et de régulation dudit gène de la Nitrate Réductase de tomate.

8. Vecteur de clonage et d'expression selon la revendication 7, caractérisé en ce qu'il comporte la séquence d'ADN du gène de la Nitrate Réductase de tomate allant de la position 232 à 483 sur la figure 1.

9. Vecteur selon l'une des revendications précédentes, caractérisé en ce qu'il comporte une séquence d'ADN choisie parmi les séquences tirées de la figure 1 suivantes :

- les séquences de la position 1 ou 569 à 5309,
- les séquences de la position 1 ou 569 à 4658,
- la séquence de la position 569 à 4658 privée des introns représentés en lettres minuscules.

10. Procédé pour améliorer l'assimilation des nitrates dans les plantes, caractérisé en ce qu'on introduit dans les cellules desdites plantes une séquence d'ADN ou un vecteur selon l'une des revendications 1 à 9, dans des conditions permettant sa réplication et son expression.

```
tacgatgaaaaatacaccttaaaatgttagtcgaagtttttgtaatttgactctgaaaatagaaaccacgacacttaatagtgaacggagagagtaattgatataatt  108

tctattttagagtcaaactataaaatttaaccaacatcttgtaatttagtagtatttttcatatataatttttgaatatctaaaatttttaactttgaaagttaacgt  216

aacatgacaaataaaatcaataaatgaggaaaataattttcatcaaatttaaaattgtttttaatatttgaacgataaaattgtaaatcatatagacgaatttttatt  324

atttttgctgatgatgtcacaaacttttgtaatcaaaattcaattttggtgtcgatttttttgggtcctacttatgtacagtaaatatggggttgagatagttcgtaac  432

[illegible]  540

cattcataattttattttaaaaaaatca ATG GCG GCA TCT GTG GAA AAC AGA CAG TAT ACT CAC CTT GAA CCG GGT TTA TCA GGC GTA  628
                              M   A   A   S   V   E   N   R   Q   Y   T   H   L   E   P   G   L   S   G   V    20

GGC CGT ACT TTC AAG CCT AGG CCT GAT TCC CCG GTT CGT GGT TGC AAC TTC CCT CCT TCA TCC AAC CAT GAA CTC CCT TTC  709
 G   R   T   F   K   P   R   P   D   S   P   V   R   G   C   N   F   P   P   S   S   N   H   E   L   P   F    47

CAA AAA AAA CAA AAT CCC CCA ATT TAC CTT GAT TAT TCG TCT AGT GAA GAC GAG GAT GAC GAT GAC GAA AAA AAT GAA TAC  790
 Q   K   K   Q   N   P   P   I   Y   L   D   Y   S   S   S   E   D   E   D   D   D   D   E   K   N   E   Y    74

GTT CAA ATG ATC AAA AAA GGT AAA ACT GAA TTA GAA CCA TCA ATT CAT GAT ACT AGA GAT GAA GGT ACC GCT GAT AAT TGG  871
 V   Q   M   I   K   K   G   K   T   E   L   E   P   S   I   H   D   T   R   D   E   G   T   A   D   N   W   101

ATC GAA CGA AAC TTT TCC TTA ATA CGT CTC ACC GGT AAA CAC CCA TTC AAC TCC GAA CCA CCT TTA TCT CGC CTT ATG CAT  952
 I   E   R   N   F   S   L   I   R   L   T   G   K   H   P   F   N   S   E   P   P   L   S   R   L   M   H   128

CAC GGG TTC ATC ACT CCC GTA CCA CTT CAT TAC GTC CGT AAC CAC GGC CCA GTC CCC AAA GCA TCC TGG TCT GAC TGG ACT 1033
 H   G   F   I   T   P   V   P   L   H   Y   V   R   N   H   G   P   V   P   K   A   S   W   S   D   W   T   155
```

FIG_1A

```
GTG GAA GTT ACA GGG CTG GTA AAA CGA CCA ATG AAA TTC ACA ATG GAT CAA TTA GTT AAC GAA TTC CCT TCA CGT GAA TTC 1114
 V   E   V   T   G   L   V   K   R   P   M   K   F   T   M   D   Q   L   V   N   E   F   P   S   R   E   F   182

CCT GTC ACA CTT GTG TGC GCA GGC AAT CGT CGT AAA GAG CAG AAT ATG GTG AAG CAG ACA ATT GGT TTC AAT TGG GGT GCT 1195
 P   V   T   L   V   C   A   G   N   R   R   K   E   Q   N   M   V   K   Q   T   I   G   F   N   W   G   A   209

GCT GCC GTT TCA ACC ACC GTA TGG CGC GGA GTA CCT CTC CGC GCC CTG TTG AAA CGG TGC GGT GTT CAG AGT AAG AAA AAA 1276
 A   A   V   S   T   T   V   W   R   G   V   P   L   R   A   L   L   K   R   C   G   V   Q   S   K   K   K   236

GGC GCG CTT AAT GTC TGT TTC GAA GGT TCC GAT GTT TTG CCT GGA GGT GGT GGT TCA AAG TAC GGA ACG AGT ATA AAG AAG 1357
 G   A   L   N   V   C   F   E   G   S   D   V   L   P   G   G   G   G   S   K   Y   G   T   S   I   K   K   263

GAA TTC GCC ATG GAT CCA TCT CGT GAT ATT ATT GTA GCT TAC ATG CAA AAC GGA GAA ATG TTG TCA CCG GAT CAT GGT TTT 1438
 E   F   A   M   D   P   S   R   D   I   I   V   A   Y   M   Q   N   G   E   M   L   S   P   D   H   G   F   290

CCG GTA AGG ATG ATT ATC CCC GGA TTC ATC GGT GGA AGA ATG GTG AAA TGG TTA AAG AGG ATT GTG GTC ACT ACA CAA GAA 1519
 P   V   R   M   I   I   P   G   F   I   G   G   R   M   V   K   W   L   K   R   I   V   V   T   T   Q   E   317

TCG GAA AGC TAT TAT CAT TAC AAG GAC AAT AGA GTC CTC CCT CCA CAC GTT GAC GCG GAA CTT GCC AAC GCG GAA Ggtacgat 1602
 S   E   S   Y   Y   H   Y   K   D   N   R   V   L   P   P   H   V   D   A   E   L   A   N   A   E            342

atgaataataattagcatctattttagatcaaagttatttattatactcaagttgtcatttgtttag CT TGG TGG TAC AAA CCA GAG TAC ATC ATC 1698
                                                                    A   W   W   Y   K   P   E   Y   I   I   352

AAT GAG CTC AAC ATA AAC TCT GTC ATT ACA ACT CCG TGC CAT GAA GAA ATT TTG CCC ATC AAT GCG TGG ACT ACT CAG AGA 1779
 N   E   L   N   I   N   S   V   I   T   T   P   C   H   E   E   I   L   P   I   N   A   W   T   T   Q   R   379
```

FIG-1B

```
CCT TAC ACG TTG AGA GGC TAT GCT TAT TCT Ggttagttatttttctctctgatttgctgaattttcttagtactgtcgaatttcatcaactgtttgtt 1877
 P   Y   T   L   R   G   Y   A   Y   S                                                                            389

ttaattatttcgtacctcttgttttcagctaatttcaacctcaccctcttatctcatattatcatacaaattttaagatttcatgaaaaagtatattagtacgaata 1985

ttttgtatgttgttcgaacttttttagaaatatcaataaatatatatcgtatttgtcgaaaatactgtatttttaaagagtctgcaatttaggatagtaaaggaaaaat 2093

aggggtgagtggaggtggatgcatgaactttttgatggtgtcatcaacttgtttggttatgtaaacagaatagcaagaaaaagacaatatagttggatttatttaatg 2201

gtttacagaaaaacctggggagcttttcctagttctgaagagtcggtctaaaaataatatataggtaacaaaatttaattgcctacaaagacagaatctttttgact 2309

gctttagttcctgcatcttaggctgcctcaacaacattataataagttaaattattattattatataatacatcattgtgtttgtattcaatttgaaaactttcaagt 2417

ggtcactttatatagatctagtcaatattattttaatttaagcctaggtataaggacttttatctttttctaatcaacgtgcctaacttgttgcaattttgattcaaa 2525

tacaagtttgacccttcaagtggggaaagtgttacatttttttaattgaatagtcagttacatgtttaaattaagctattagacacgattacagttgaatttatgaatt 2633

gacgagatggtgattgtgtaatag GT GGA GGT AAA AAG GTA ACT CGA GTG GAA GTG ACT TTG GAT GGA GGA GAG ACA TGG AGT GTG  2719
                         G   G   G   K   K   V   T   R   V   E   V   T   L   D   G   G   E   T   W   S   V    410

TGT ACA CTT GAT CAC CCA GAG AAG CCA ACA AAG TAT GGC AAG TAC TGG TGT TGG TGC TTT TGG TCA CTC GAG GTT GAG GTG  2800
 C   T   L   D   H   P   E   K   P   T   K   Y   G   K   Y   W   C   W   C   F   W   S   L   E   V   E   V    437
```

FIG_1C

```
CTT GAC TTG CTT AGT GCT AAA GAA ATT GCT GTA CGA GCT ACC GAT GAG ACC CTC AAC ACT CAA CCC GAG AAG CTT ATT TGG  2881
 L   D   L   L   S   A   K   E   I   A   V   R   A   T   D   E   T   L   N   T   Q   P   E   K   L   I   W    464

AAC GTC ATG gtaaattcacatttaactttttacaacttctttaaaattaatggcgtaaagtcaaatatatacatgtaactagtccttgatgaaaagtaattttca 2986
 N   V   M                                                                                                      467

gtccttttcaacttttcttttatttatgattactttaccatatgcagattgatagagcatgtgatttcacataaaaacaaatttttttcattggtttaagtttcagat 3094

[illegible]ttcaaaaaaagaaagtaatgccaattcctattatgcgtaagcagcagcgtgatcacatgaatatgtcacg 3202

tgctaggcctcttattataatttaagttgatcgtgttactgatggaggacggttactagtaagtaactggtaatgttgttatacatttgtctaattttatggtgatgg 3310

atgttgtaatgttcag GGA ATG ATG AAC AAT TGT TGG TTT CGA GTG AAG ATG AAT GTG TGC AAA CCT CAC AAG GGA GAG ATT GGT 3395
                  G   M   M   N   N   C   W   F   R   V   K   M   N   V   C   K   P   H   K   G   E   I   G    490

ATA GTG TTT GAG CAT CCG ACT CAA CCT GGA AAT CAA TCG GGT GGA TGG ATG GCA AAG GAG AGA CAC TTG GAG ATA TCA GCA  3476
 I   V   F   E   H   P   T   Q   P   G   N   Q   S   G   G   W   M   A   K   E   R   H   L   E   I   S   A    517

GTG GCT CCT CCA ACA CTA AAG AAG AGT ATA TCA ACT CCT TTC ATG AAC ACA GCT TCG AAG ATG TAT TCC ATG TCC GAG GTG  3557
 V   A   P   P   T   L   K   K   S   I   S   T   P   F   M   N   T   A   S   K   M   Y   S   M   S   E   V    544

AGG AAA CAC AAC TCT TCA GAC TCT GCT TGG ATC ATA GTC CAT GGA CAT ATC TAC GAT GCC TCA CGT TTC TTG AAA GAC CAT  3638
 R   K   H   N   S   S   D   S   A   W   I   I   V   H   G   H   I   Y   D   A   S   R   F   L   K   D   H    571

CCC GGT GGT GTT GAC AGC ATT CTG ATC AAT GCT GGA ACT GAT TGT ACT GAG GAA TTT GAT GCA ATT CAT TCT GAT AAG GCT  3719
 P   G   G   V   D   S   I   L   I   N   A   G   T   D   C   T   E   E   F   D   A   I   H   S   D   K   A    598
```

FIG_1D

```
AAG AAG CTA TTG GAG GAC TTT AGG ATT GGT GAA CTC ATA ACT ACT GGT TAC ACG TCT GAT TCG TCT CCA AAC AGT TCT GTC  3800
 K   K   L   L   E   D   F   R   I   G   E   L   I   T   T   G   Y   T   S   D   S   S   P   N   S   S   V    625

CAT GGA TCC TCT TCG ATC AGT AGC TTC TTA GCA CCT ATT AAG GAG CTT GTT CAA ACA CCA ACA AGG AGT GTA GCT CTC ATC  3881
 H   G   S   S   S   I   S   S   F   L   A   P   I   K   E   L   V   Q   T   P   T   R   S   V   A   L   I    652

CCA AGG GAA AAA ATC CCT TGC AAA CTC GTC GAC AAG CAA TCC ATC TCC CAT GAT GTT AGG AAA TTC AAA TTT GCA TTA CCC  3962
 P   R   E   K   I   P   C   K   L   V   D   K   Q   S   I   S   H   D   V   R   K   F   K   F   A   L   P    679

TCT GAG GAT CAA GTC TTA GGG TTA CCT GTT GGC AAA CAC ATA TTC CTC TGT GCC ACA GTT GAT GAC AAA CTC TGT ATG CGT  4043
 S   E   D   Q   V   L   G   L   P   V   G   K   H   I   F   L   C   A   T   V   D   D   K   L   C   M   R    706

GCC TAC ACG CCT ACT AGC ACA GTC GAT GAA GTA GGG TTC TTC GAG TTG GTT GTC AAG ATC TAC TTC AAA GGT GTT CAC CCT  4124
 A   Y   T   P   T   S   T   V   D   E   V   G   F   F   E   L   V   V   K   I   Y   F   K   G   V   H   P    733

AAA TTC CCT AAT GGA GGT CAA ATG TCA CAA CAT CTT GAT TCT CTC CCA ATA GGT GCA TTC CTT GAC GTT AAA GGT CCA TTA  4205
 K   F   P   N   G   G   Q   M   S   Q   H   L   D   S   L   P   I   G   A   F   L   D   V   K   G   P   L    760

GGT CAC ATT GAA TAC CAA GGT AAG GGT AAT TTC TTA GTC CAT GGT AAA CAA AAG TTT GCC AAG AAG TTA GCT ATG ATA GCG  4286
 G   H   I   E   Y   Q   G   K   G   N   F   L   V   H   G   K   Q   K   F   A   K   K   L   A   M   I   A    787

GGT GGA ACA GGT ATA ACT CCA GTA TAT CAA GTA ATG CAA TCA ATA TTG AAA GAT CCT GAA GAC GAT ACA GAG ATG TAT GTG  4367
 G   G   T   G   I   T   P   V   Y   Q   V   M   Q   S   I   L   K   D   P   E   D   D   T   E   M   Y   V    814

GTG TAT GCA AAC AGA ACG GAG GAT GAT ATT TTG CTC AAA GAC GAA CTT GAT GCA TGG GCA GAG CAA GTT CCA AAT AGG GTT  4448
 V   Y   A   N   R   T   E   D   D   I   L   L   K   D   E   L   D   A   W   A   E   Q   V   P   N   R   V    841
```

FIG_1E

```
AAA GTA TGG TAT GTC GTT CAA GAA TCC ATT ACA CAA GGA TGG AAG TAT AGT ACA GGA TTC GTT ACA GAA TCG ATT CTT AGA  4529
 K   V   W   Y   V   V   Q   E   S   I   T   Q   G   W   K   Y   S   T   G   F   V   T   E   S   I   L   R    868

GAA CAT ATA CCT GAA CCA TCT CAT ACA ACA TTG GCA TTA GCA TGT GGA CCA CCT CCA ATG ATA CAA TTT GCT ATT AAT CCA  4610
 E   H   I   P   E   P   S   H   T   T   L   A   L   A   C   G   P   P   P   M   I   Q   F   A   I   N   P    895

AAC TTG GAG AAA ATG GGA TAT GAC ATT AAG GAG GAA CTA TTG GTG TTC taaattggatggtgatgatgatagatgatatatctctttggagg 4702
 N   L   E   K   M   G   Y   D   I   K   E   E   L   L   V   F                                                911

aataaattcttttgtattttcagttgtacatattgtgtttttgtttatcatcaaaatgtactacttcttgtagttcttacattttaattttctactcaactttaggta 4810

taaaatgttgtactgtgtactagtatgtgttatgtcaaagttaagattgtattcatcatggaattacagtactttgttgtcacaagtcttgttgtatatatttttacc 4918

aaattaatgtgtatatatatatagtaaattgaaacatttgtgtgtgtgtttatttatttcctctcaactctaaaactacaatttttttgctcttcattcaatatta 5026

[illegible] 5134

taataataaaaatatgtttttttacaaaagtttaagtctttagatcacatggtcacacaattcaatatgatatcagaactgacagaagttttgaaatcgaattatcag 5242

ttagtcgagtaatcttattttgctatattttatatatacctatagtgttaaacttcacgaaaagaca                                        5309
```

FIG.1F

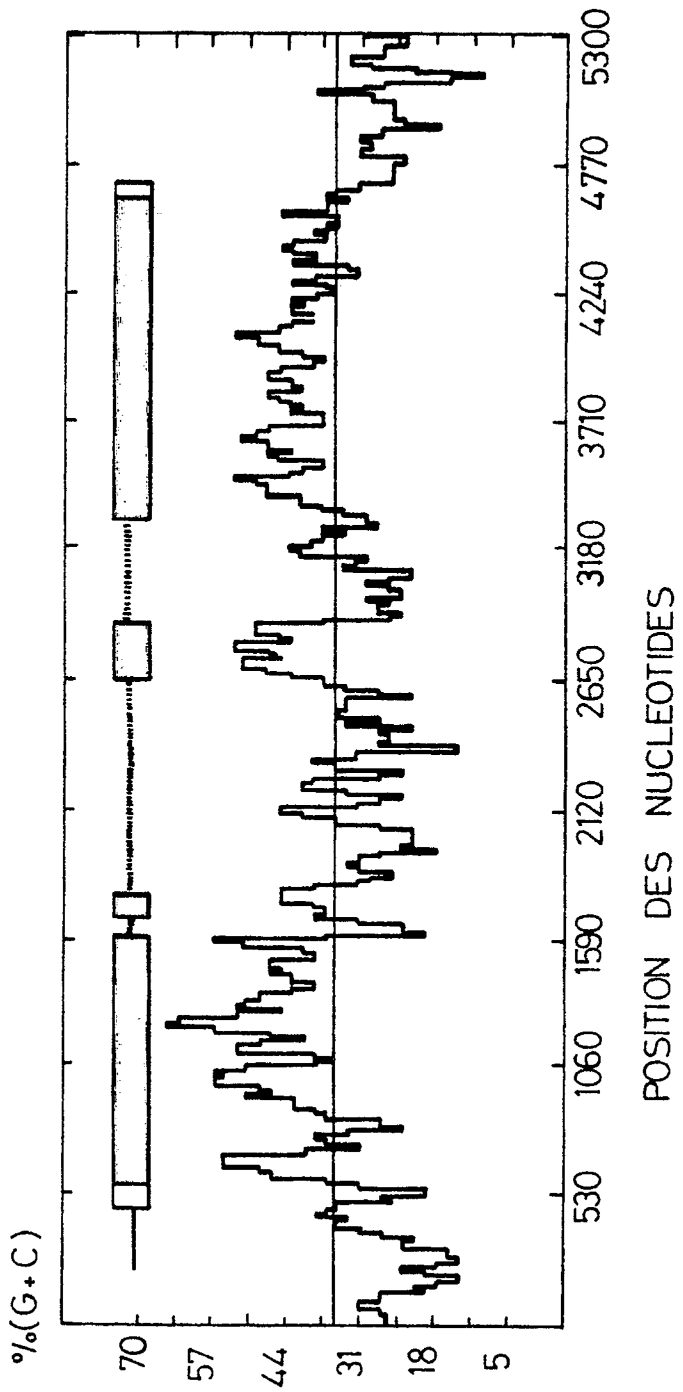
%(G+C)
70
57
44
31
18
5
530
1060
1590
2120
2650
3180
3710
4240
4770
5300
POSITION DES NUCLEOTIDES

FIG_2

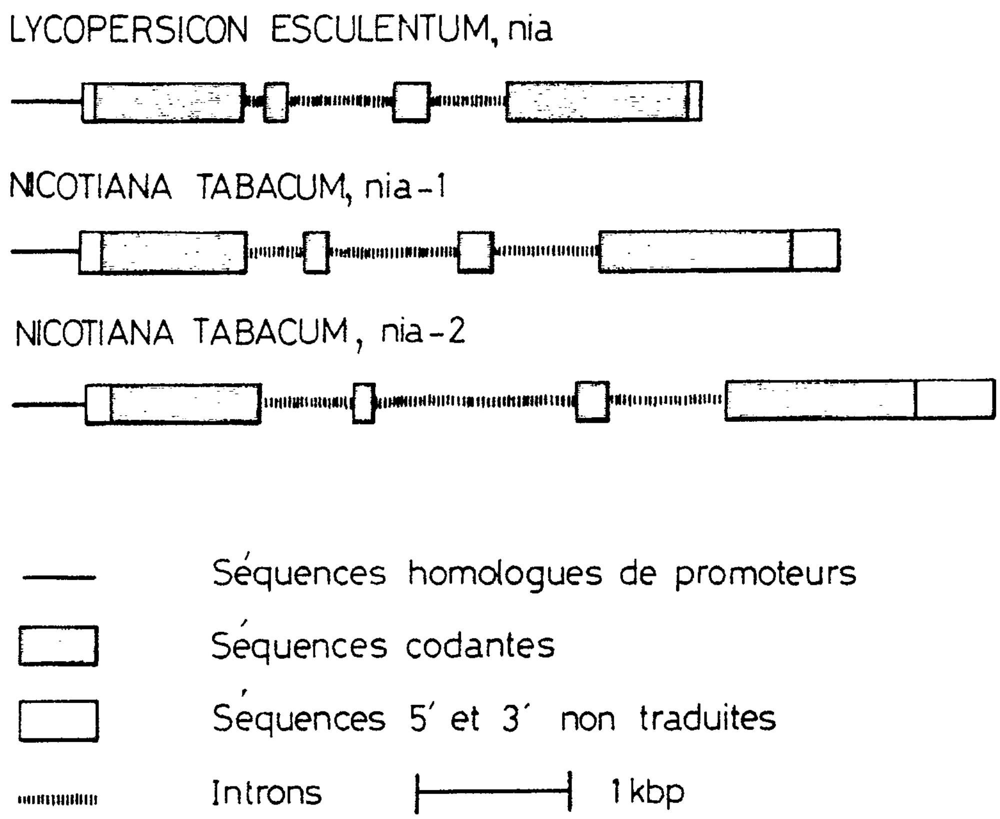
LYCOPERSICON ESCULENTUM, nia
NICOTIANA TABACUM, nia-1
NICOTIANA TABACUM, nia-2
Séquences homologues de promoteurs
Séquences codantes
Séquences 5′ et 3′ non traduites
Introns
1 kbp

FIG_3

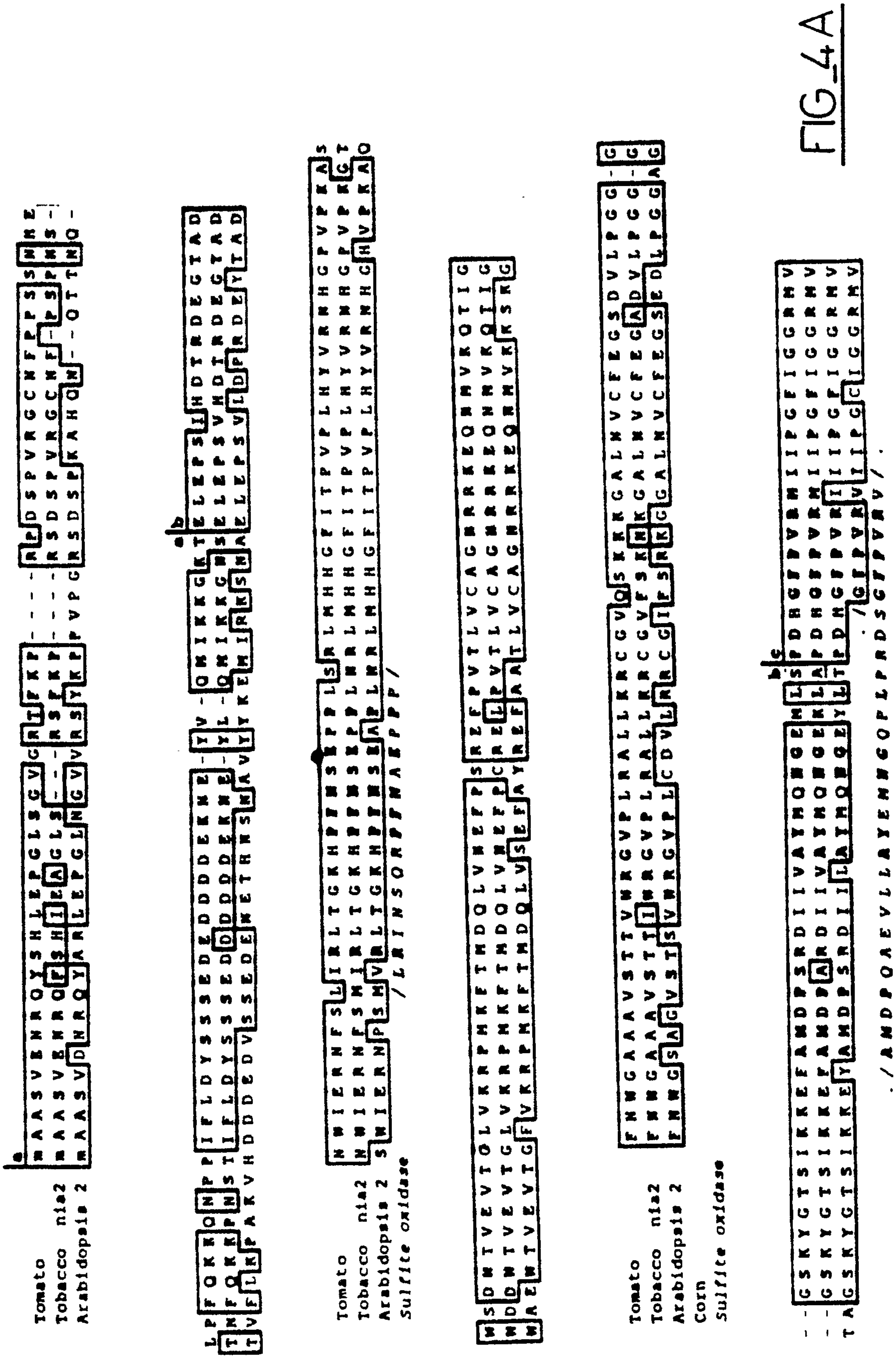


FIG_4A

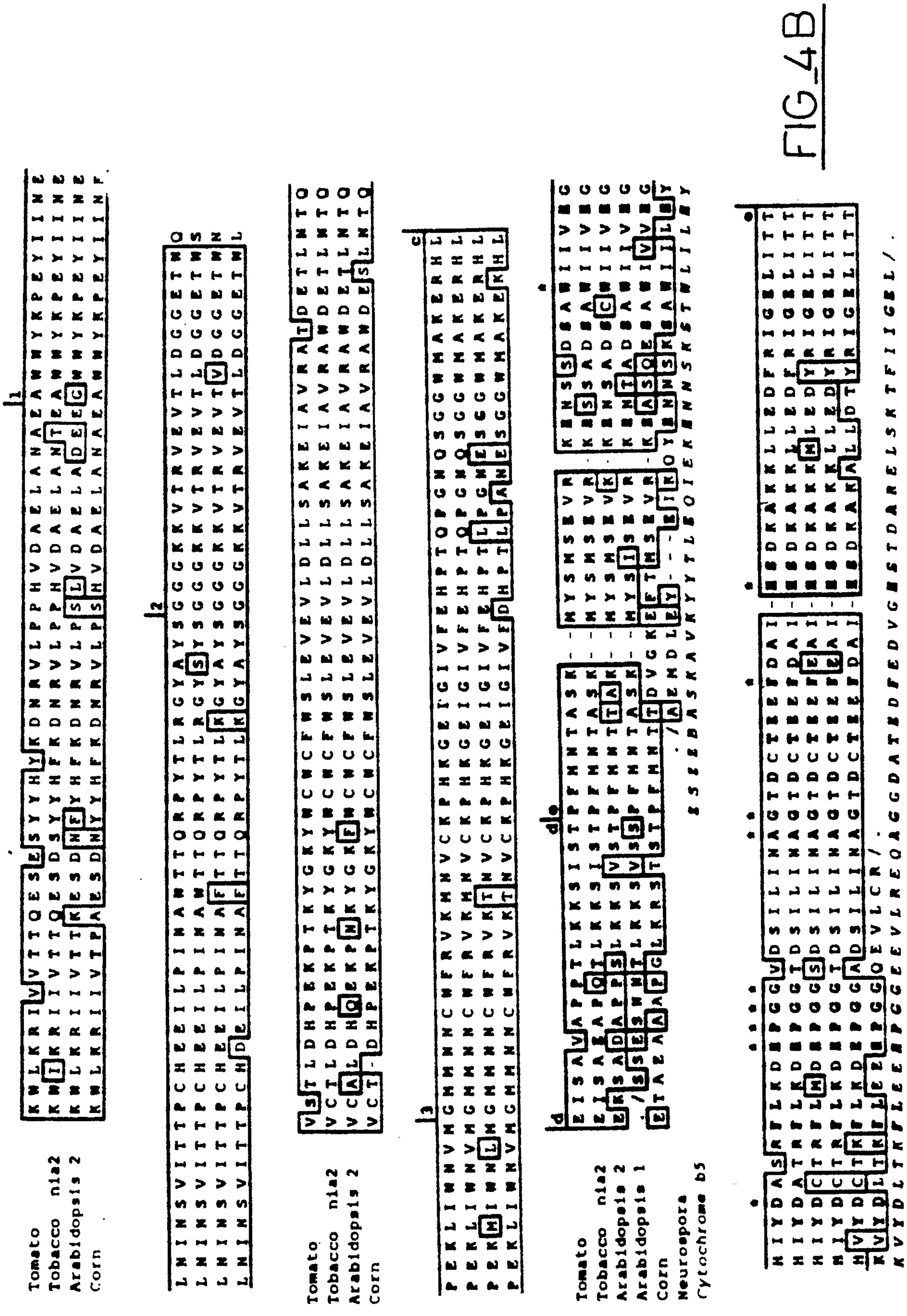

FIG_4B

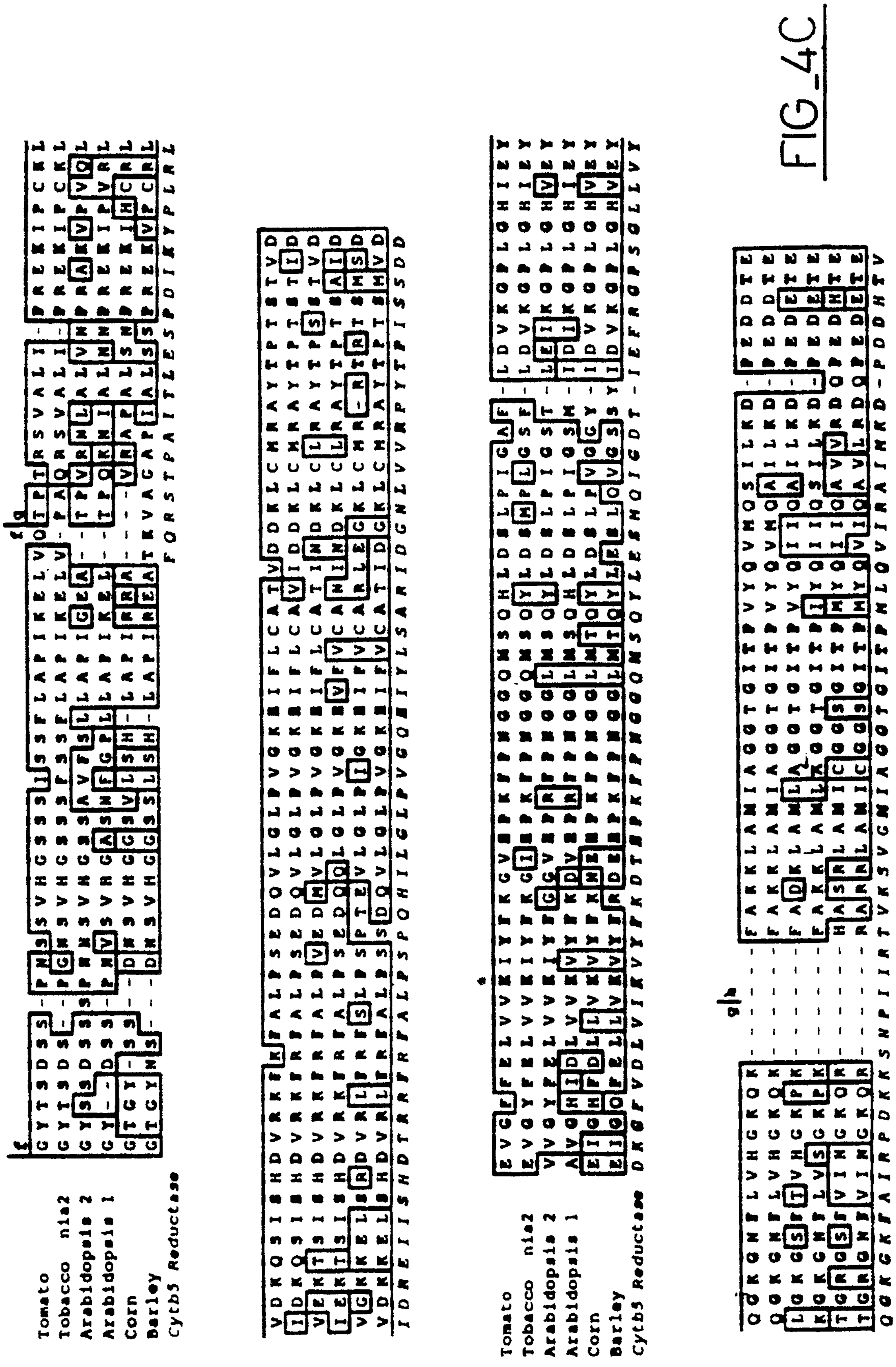
Tomato
Tobacco nia2
Arabidopsis 2
Arabidopsis 1
Corn
Barley
Cytb5 Reductase

FIG_4C

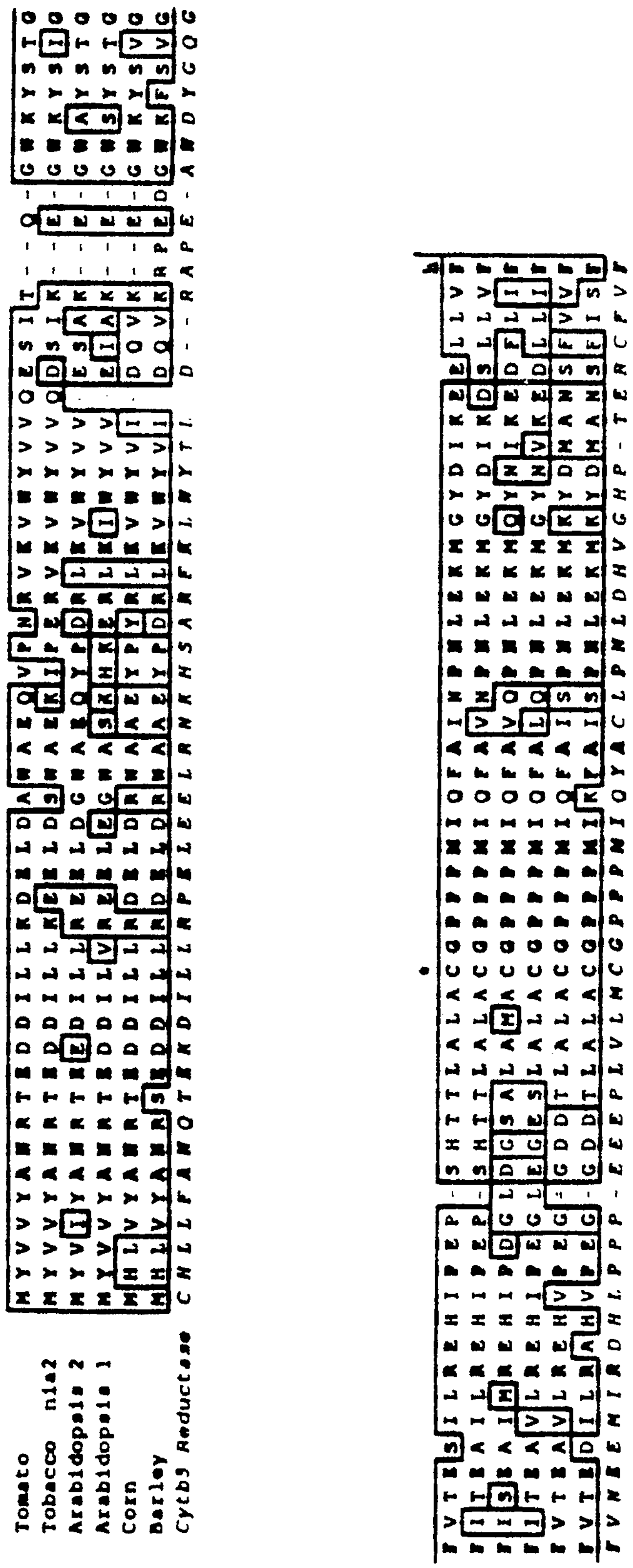
Tomato
Tobacco nia2
Arabidopsis 2
Arabidopsis 1
Corn
Barley
Cytb5 Reductase

FIG_4D

Office européen des brevets

# RAPPORT DE RECHERCHE EUROPEENNE

Numero de la demande

EP 90 40 2077

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | CHEMICAL ABSTRACTS, vol. 109, 1988, page 485, résumé no. 226806y, Columbus, Ohio, US; F. GALANGAU et al.: "Expression of leaf nitrate reductase genes from tomato and tobacco in relation to light-dark regimes and nitrate supply" * Abrégé * --- | 1-10 | C 12 N 15/53<br>C 12 N 15/82 |
| O,A | JOURNAL OF CELLULAR BIOCHEMSTRY, suppl. 13D, page 292, abrégé M258 & 18TH ANNUAL UCLA SYMPOSIA PLANT GENE TRANSFER, 1-7 avril 1989, Park City, Utah, US; H. VAUCHERET et al.: "Molecular genetics of tobacco nitrate reductase" * Abrégé * --- | 1-10 | |
| A | PLANT MOLECULAR BIOLOGY, vol. 12, mai 1989, pages 597-600, Kluwer Academic Publishers, BE; H. VAUCHERET et al.: "Complete nucleotide sequence of the two homeologous tobacco nitrate reductase genes" * En entier * --- | 1-10 | **DOMAINES TECHNIQUES RECHERCHES (Int. Cl.5)**<br>C 12 N |
| A | MOL. GEN. GENET., vol. 216, mars 1989, pages 10-15, Berlin, DE; H. VAUCHERET et al.: "Molecular cloning and characterisation of the two homeologous genes coding for nitrate reductase in tobacco" * En entier * --- -/- | 1-10 | |

Le présent rapport a été établi pour toutes les revendications

| Lieu de la recherche | Date d'achèvement de la recherche | Examinateur |
|---|---|---|
| LA HAYE | 03-10-1990 | MADDOX A.D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 90 40 2077

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | MOL. GEN. GENET., vol. 209, 1987, pages 552-562, Springer Verlag; R. CALZA et al.: "Cloning of DNA fragments complementary to tobacco nitrate reductase mRNA and encoding epitopes common to the nitrate reductases from higher plants" * En entier * --- | 1-10 | |
| A | EP-A-0 227 909 (GENERAL HOSPITAL CORP.) * Page 2, lignes 45-54; page 3, lignes 10-20 * --- | 10 | |
| P,X | GENE, vol. 85, 1989, pages 371-380, Elsevier Science Publishers B.V. (Biomedical Division); F. DANIEL-VEDELE et al.: "Cloning and analysis of the tomato nitrate reductase-encoding gene: protein domain structure and amino acid homologies in higher plants" * En entier * --- | 1-9 | DOMAINES TECHNIQUES RECHERCHES (Int. Cl.5) |
| A | EP-A-0 283 338 (INRA) * Page 2, lignes 12-20 * ----- | 10 | |

Le présent rapport a été établi pour toutes les revendications

| Lieu de la recherche | Date d'achèvement de la recherche | Examinateur |
|---|---|---|
| LA HAYE | 03-10-1990 | MADDOX A.D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)